Europäisches Patentamt

European Patent Office

Office européen des brevets

(19)

(11) Numéro de publication: **0 251 843**
**A1**

## (12) DEMANDE DE BREVET EUROPEEN

(21) Numéro de dépôt: 87401255.2

(22) Date de dépôt: 04.06.87

(51) Int. Cl.³: **C 12 N 15/00**
C 12 P 21/02, C 12 N 5/00
C 07 K 13/00, A 61 K 37/02

(30) Priorité: 06.06.86 FR 8608258
03.04.87 FR 8704699

(43) Date de publication de la demande:
07.01.88 Bulletin 88/1

(84) Etats contractants désignés:
AT BE CH DE ES FR GB GR IT LI LU NL SE

(71) Demandeur: TRANSGENE S.A.
16, rue Henri Régnault
F-92400 Courbevoie(FR)

(72) Inventeur: Pavirani, Andrea
4, rue Stimmer
F-67000 Strasbourg(FR)

(72) Inventeur: Meulien, Pierre
73, avenue des Vosges
F-67000 Strasbourg(FR)

(72) Inventeur: Lecocq, Jean-Pierre
6, rue du Champ du Feu
F-67116 Reichstett(FR)

(74) Mandataire: Ahner, Francis et al,
CABINET REGIMBEAU 26, avenue Kléber
F-75116 Paris(FR)

(54) Procédé de préparation de facteur VIII à partir de cellules de mammifères.

(57) L'invention concerne un procédé de préparation de facteur VIII, caractérisé en ce qu'on met en culture, dans un milieu de culture approprié, des cellules de mammifères infectées par un vecteur assurant l'expression du facteur VIII, ledit milieu contenant le facteur von Willebrand.

La présente invention concerne en outre ledit vecteur comportant une séquence codant pour au moins l'un des polypeptides, la chaîne lourde du facteur VIII, HC ou la chaîne légère du facteur VIII, LC.

## PROCEDE DE PREPARATION DE FACTEUR VIII A PARTIR DE CELLULES DE MAMMIFERES

La présente invention concerne de nouveaux vecteurs d'expression du facteur VIII humain ainsi qu'un procédé pour la préparation du facteur VIII.

Le clonage récent du facteur VIII (FVIII) ou facteur anti-hémophilique (réf. 1 à 3 et 29) permet d'espérer, pour les patients hémophiles A, une thérapeutique plus sure.

Les patients hémophiles A sont traités avec des concentrés de plasma de FVIII contenant moins de 0,1 % d'activité pro-coagulante de FVIII (réf. 4) qui sont obtenus à partir de mélanges de plasma provenant de nombreux donneurs.

Les hémophiles sont, dans ces conditions, soumis à des risques très importants de contagion par des agents pathogènes tels que le virus de l'hépatite ou le virus du LAV/HTLVIII, l'agent causatif du SIDA.

En utilisant des techniques de génie génétique, il est possible de préparer du FVIII recombinant, lequel sera produit en quantités très importantes et évidemment exempt de contamination, ce qui le rendrait beaucoup plus acceptable sur le plan thérapeutique.

Le rendement et la stabilité du produit recombinant, toutefois, dépendent dans une certaine mesure du système d'hôte utilisé pour l'expression.

Des mécanismes particuliers sont responsables du rendement ou du maintien de l'intégrité de la protéine exprimée, par exemple des modifications post-traductionnelles correctes. Un système test est intéressant pour évaluer les différentes lignées cellulaires et vérifier, en particulier, la conformité de la protéine obtenue. Les systèmes basés sur le virus de la vaccine sont, de ce fait, extrêmement puissants car, tout à la fois, le virus sauvage et le virus recombinant peuvent infecter et se répliquer dans pratiquement un nombre illimité de cellules. En outre, ce système ne nécessite pas l'utilisation de système de sélection de longue durée comme les lignées cellulaires hôtes présentant un marqueur, ce qui simplifie grandement son utilisation.

L'expression d'une séquence codante pour une protéine exogène dans le virus de la vaccine (VV) implique nécessairement deux étapes :

1) La séquence codante doit être sous la dépendance d'un promoteur de VV et être insérée dans un segment non essentiel de l'ADN de VV, cloné dans un plasmide bactérien approprié.

2) Les séquences d'ADN de VV situées de part et d'autre doivent permettre des recombinaisons homologues <u>in vivo</u> entre le plasmide et le génome viral. Une double recombinaison réciproque conduit à un transfert de l'insert d'ADN du plasmide dans le génome viral par lequel il sera propagé et exprimé (réf. 5 : Smith et col., 1983 ; 6 : Panicali et col., 1983 ; 7 : Kieny et col., 1984).

La présente invention concerne un poxvirus, en particulier un virus de la vaccine, caractérisé en ce qu'il comporte la séquence d'ADN codant pour le facteur VIII. Ce virus comportera, de préférence, l'ensemble des éléments assurant l'expression du FVIII mature complet qui sera sécrété par les cellules dans le milieu.

3

En particulier, il comportera un promoteur d'un gène de poxvirus, notamment de la vaccine, situé en amont de la séquence codant pour le FVIII, tel que le promoteur du gène de 7,5K, noté P7,5K, qui assurera la transcription de la séquence d'ADN codant pour FVIII, nommée ci-après : d'ADN(FVIII).

Cet ensemble promoteur/séquence d'ADN(FVIII) sera inséré dans une séquence non essentielle de l'ADN de la vaccine telle qu'un gène du virus de la vaccine, par exemple le gène TK (thymidine kinase), ce qui fournit une possibilité de sélection, comme cela sera expliqué ci-après.

Bien entendu, dans le cadre de la présente invention, lorsque l'on parle de "virus de la vaccine", il peut s'agir du virus entier ou d'un virus dont certaines parties non essentielles ont été délétées.

La présente invention s'étend aux vecteurs comportant une séquence codant pour au moins l'un des polypeptides choisi parmi la chaîne lourde et la chaîne légère du facteur VIII.

En effet, la purification de la molécule naturelle du facteur VIII (23 à 28) et le clonage du gène correspondant ont apporté des informations sur la structure et sur la fonction du facteur VIII et ont permis de déterminer la position des sites de clivage responsables de l'activation de la molécule.

Un premier clivage (par une protéase non identifiée) se produit entre les acides aminés 1648 et 1649 et entraîne la dégradation protéolytique de la partie centrale de la molécule (domaine B) ; ensuite la thrombine clive la partie $NH_2$-terminale à l'acide aminé 740. Il en résulte deux polypeptides d'environ 90 kd et 80 kd qui sont maintenus ensemble par un pont calcium.

Il a été démontré (23 à 28) que ces deux polypeptides (chaîne lourde : HC et chaîne légère : LC) sont nécessaires et suffisants pour donner l'activité procoagulante du facteur VIII (FVIII:C), en effet, les sites d'activation par la thrombine sont situés à l'acide aminé 372 sur la chaîne lourde et 1689 sur la chaîne légère. Cette observation suggère que le domaine B n'est pas nécessaire à la fonction procoagulante de la molécule.

La présente invention concerne plus particulièrement le clonage des séquences codantes des deux polypeptides HC et LC séparément, en utilisant le virus de la vaccine comme vecteur, et l'expression simultanée des deux molécules par co-infection de cellules de mammifères avec les deux virus recombinants.

Ainsi, le vecteur préféré selon la présente invention est caractérisé en ce que la séquence codant pour une partie du FVIII code pour au moins l'un des polypeptides, la chaîne lourde du FVIII, HC ou la chaîne légère du FVIII, LC. De préférence, la séquence codant pour HC ou LC est précédée de la séquence signal du facteur VIII.

La présente invention concerne également les cellules de mammifères infectées par un vecteur tel que décrit précédemment, ainsi que les plasmides utiles pour la construction desdits vecteurs et comportant au moins une séquence codant pour HC ou LC.

Parmi les cellules particulièrement utilisables, il faut citer notamment les cellules BHK, de préférence BHK21 ; mais d'autres cellules peuvent être utilisées, comme cela sera décrit dans les exemples.

5

Ces cellules infectées et mises en culture, qu'elles aient été transfectées par un vecteur codant pour HC ou LC ou bien co-transfectées par deux vecteurs codant, l'un pour HC, l'autre pour LC, conduisent à la sécrétion de polypeptides présentant un intérêt thérapeutique.

En particulier, il est possible de prévoir la production d'une molécule ayant l'activité du facteur VIII par des cellules de mammifères par réassociation _in vivo_ des deux chaînes lourde et légère synthétisées par les deux vecteurs indépendants codant, l'un pour HC, l'autre pour LC.

Outre l'utilisation du composé obtenu par réassociation des chaînes HC et LC, il est possible de prévoir d'autres utilisations des polypeptides selon l'invention.

En effet, on sait que 8 à 20 % des patients atteints d'hémophilie A sévère et qui nécessitent des transfusions multiples développent des anticorps contre le facteur VIII. Le traitement de tels "patients inhibiteurs" par injection de concentrés de facteur VIII présente d'importantes difficultés.

Les épitopes reconnus par ces anticorps sont limités à certaines parties de la molécule. On peut envisager l'utilisation thérapeutique des portions de facteur VIII telles que décrites précédemment comme agents bloquants des anticorps ; ces portions pourraient être administrées avant l'injection du facteur VIII intact, afin de faciliter le traitement des patients inhibiteurs.

6

L'invention concerne également un procédé de préparation de FVIII   obtenu par culture des cellules décrites précédemment dans un milieu de culture approprié.

L'invention concerne, en particulier, un procédé de préparation de FVIII   dans lequel on met en culture dans un milieu de culture approprié des cellules de mammifères infectées par un vecteur assurant l'expression du facteur VIII, ledit milieu contenant le facteur von Willebrand (vWf).

Les expériences ont en effet démontré que la présence de ce facteur dans le milieu de culture permettait de stabiliser le FVIII   produit. De préférence, le facteur von Willebrand est ajouté à une concentration supérieure à 0,1 U/ml.

L'invention concerne, enfin, la protéine FVIII humaine obtenue par la mise en oeuvre dudit procédé, ainsi que la protéine FVIII sous forme mature et glycosylée obtenue à partir de culture cellulaire.

L'invention concerne, en particulier, les compositions thérapeutiques contenant à titre de principe actif le FVIII obtenu par la mise en oeuvre du procédé selon l'invention.

La présente invention s'étend aux polypeptides ayant la structure de HC ou de LC ou correspondant à la réassociation de HC + LC ainsi qu'aux compositions thérapeutiques en résultant.

Les exemples suivants illustrent les propriétés des différents  composants obtenus.

Les différents matériels mis en oeuvre sont identifiés dans les exemples.

Sauf indication contraire, les enzymes sont utilisées dans les conditions préconisées par le fabricant et les techniques mises en oeuvre sont connues de l'homme de métier.

Sur les figures :

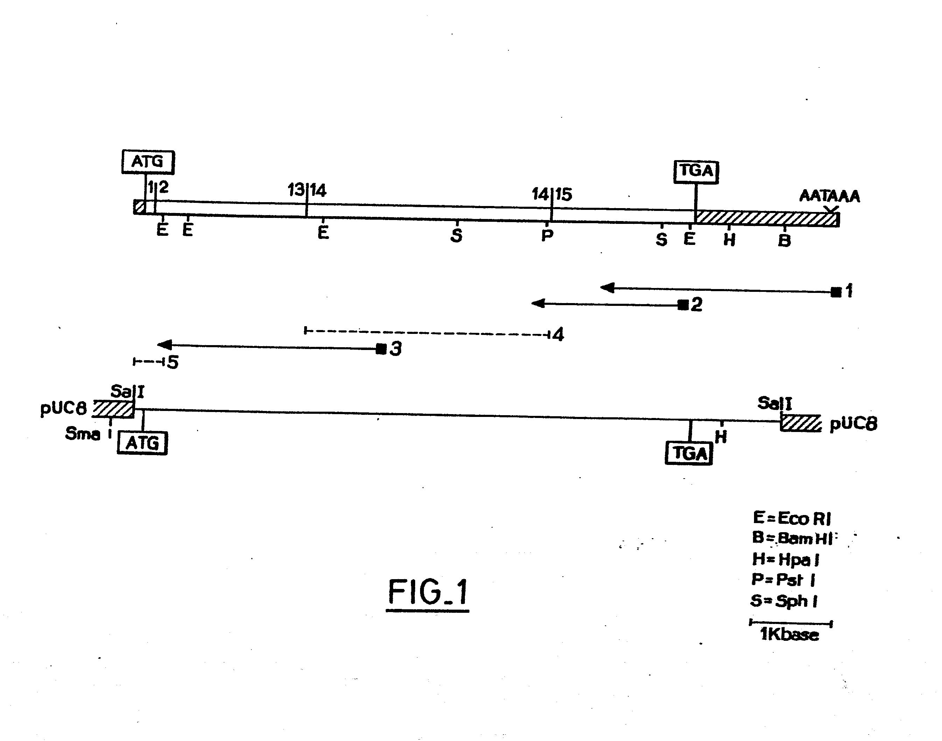

. la figure 1 schématise la préparation du gène du FVIII utilisé dans les exemples,

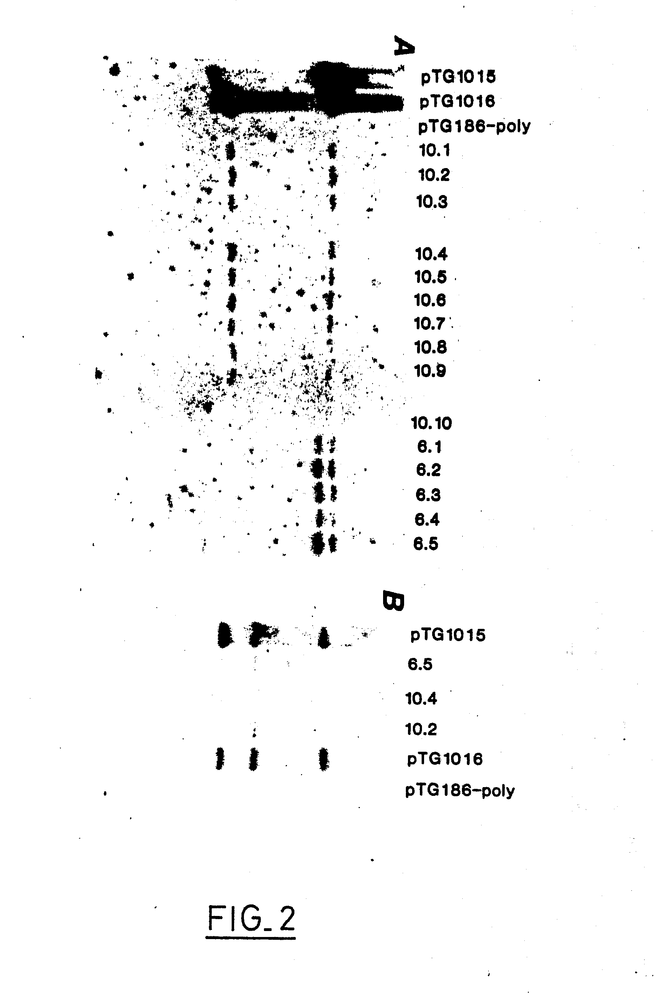

. la figure 2 est une analyse par hybridation Southern des recombinants VV contenant le FVIII recombinant, et

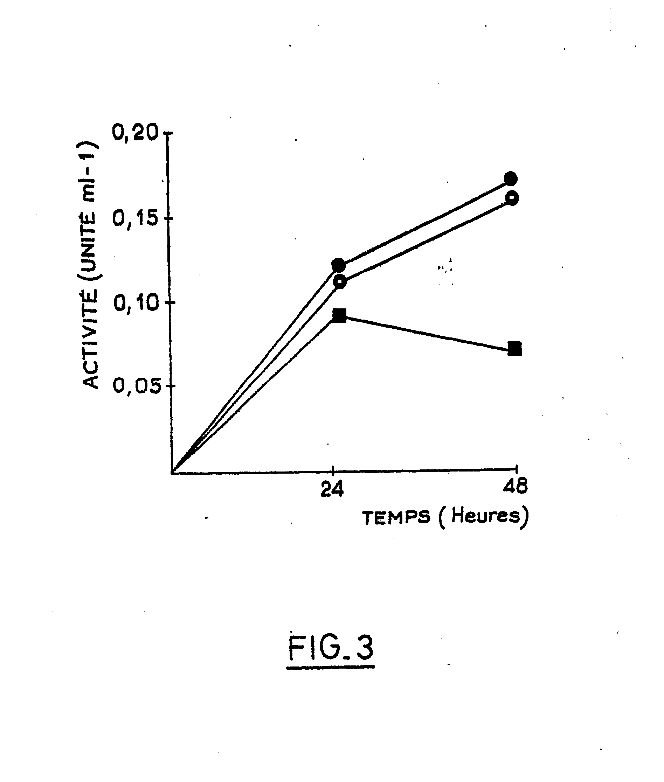

. la figure 3 schématise l'activité pro-coagulante de FVIII recombinant en fonction du temps, en présence ou en l'absence de vWf.

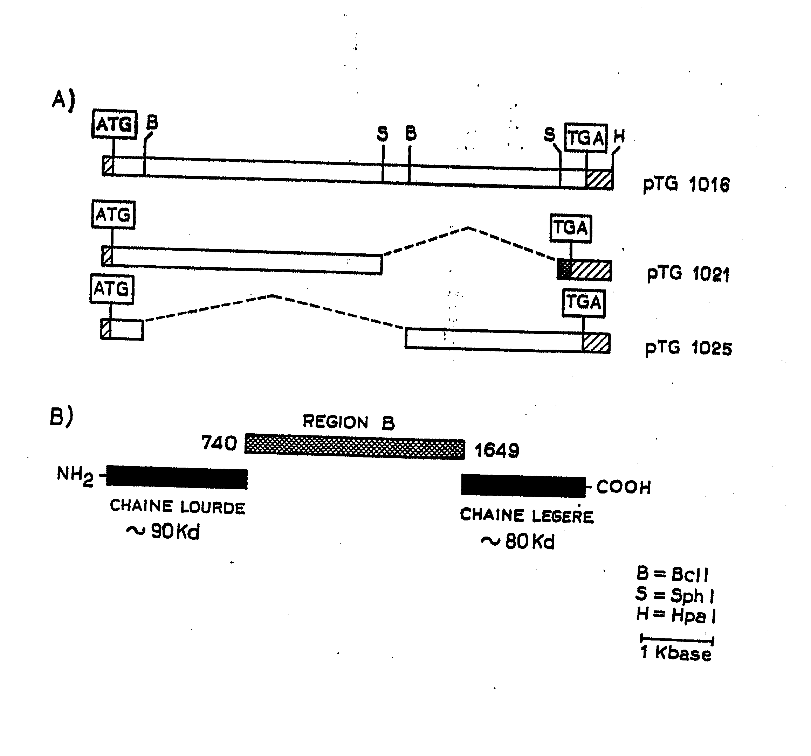

. la figure 4 schématise :

A. Représentation schématique de la construction des 2 plasmides contenant soit la chaîne lourde (Heavy chain), pTG1021, soit la chaîne légère (light chain), pTG1025, de la séquence de cADN du facteur VIII. La première ligne représente la séquence complète du cADN FVIII. Les séquences 5' et 3' non traduites sont hachurées ; le codon initiateur ATG et stop TGA et les sites de restriction utilisés pour les constructions sont indiqués.

Dans la construction pTG1021, la zone quadrillée représente la séquence décalée et le codon STOP qui sont créés par "frameshift" au niveau du site SphI

B. Représentation des produits de la première protéolyse (avant activation). Les chiffres indiquent les acides aminés où s'effectuent les clivages.

## EXEMPLE 1
## CLONAGE DE LA SEQUENCE CODANT POUR LE FACTEUR VIII

Compte tenu du fait que le mARN codant pour le FVIII humain est connu pour être extrêmement long, c'est-à-dire environ 9 000 nucléotides (ntd) (réf. 1 à 3), son clonage sous forme d'une molécule de cADN complet en utilisant la méthode classique d'amorçage à l'oligo(dT) est considéré comme non réaliste.

C'est pourquoi, sur la base des séquences de cADN déjà publiées, on a mis au point une stratégie utilisant deux chemins parallèles :

a) l'amorçage spécifique des mARN sur l'ensemble de la séquence codante, et

b) le clonage d'ADN génomique.

Des oligonucléotides synthétiques (18 et 21 mères) ont été utilisés, à la fois comme amorce pour l'élongation du cADN et comme sonde pour la détection des séquences clonées.

On a pu mettre en évidence que le criblage des banques génomiques avec deux 21 mères placés en tandem constituait une méthode très efficace pour la sélection des clones FVIII, aucun faux positif n'a été mis en évidence après analyse des séquences.

Comme cela ressort de la figure 1, des portions de cADN ont été synthétisées à partir de mARN isolés de foies humains en utilisant soit des amorces spécifiques soit de l'oligo(dT).

Comme les mARN de FVIII sont présents en très faible quantité dans ce tissu (1 mARN pour 100 000 à 200 000 molécules du mARN total) on a utilisé un vecteur de clonage du type lambdagt10 (lambdaNM607) (réf. 8) qui assure une banque représentative pouvant comporter jusqu'à 50 000 recombinants par nanogramme de cADN double brin.

Les étapes classiques de méthylation et d'addition de "linker" qui sont en général peu satisfaisantes ont été

9

écartées en utilisant une méthode "tailing-adaptor" (réf. 9).

La portion centrale de la séquence codante, qui constitue l'exon le plus grand du gène du FVIII humain, c'est-à-dire l'exon 14 dont la longueur est de 3 106 pb (réf. 10), a été clonée à partir d'une banque d'ADN génomique de lymphocytes humains. La portion 5' comportant une région non traduite 5' (UT :"untranslated"), exons 1 et 2, a été isolée sous forme de deux clones génomiques indépendants à partir d'une banque d'ADN GM1202A humain (lignée cellulaire 4XY).

De façon à assembler la séquence codante complète (voir figure 1), la région UT 5' plus le premier exon ont été accolés au second exon par une excision très précise des séquences intervenantes (un intron de 22,9 kb sépare et 2 dans le gène du FVIII (voir figure 1)).

Les autres cADN ou les clones génomiques ont alors été ligués ensemble en utilisant des sites de restriction placés de façon connue.

La construction finale de la séquence codante du FVIII introduite dans le plasmide vecteur pUC8 est appelée pTG1080 et comporte la séquence codante entière du FVIII plus la portion 5' UT (à partir de ntd -64 jusqu'à ntd -1) et la région 3' UT (à partir de ntd 7055 jusqu'à ntd 8235). La numération des ntd est effectuée selon Wood et al. (réf. 1). L'insert complet de 8,3 kb est flanqué de deux sites SalI.

De façon plus précise, l'ARN total a été extrait de foies humains adultes en utilisant le procédé au guanidinium HCl (réf. 11).

Le polyA(+)-ARN obtenu après 2 passages sur oligo(dT)-cellulose (réf. 12) a été utilisé comme support pour la synthèse du cADN. Les amorces ont été : oligo(dT) 12-18 pour le cADN 1, un 18 mère (ntd 6937 à 6954) pour le cADN 2 et un 21 mère (ntd 3067 à 3087) pour le cADN 3.

10

La synthèse du premier et du second brin, le traitement à la nucléase $S_1$ et l'enrichissement sur gradient de sucrose pour les séquences supérieures à 500 ntd ont été effectués comme cela a été décrit précédemment (réf. 9).

Toutefois, dans le cas de l'amorçage spécifique, on a trouvé qu'il était nécessaire d'utiliser au moins 50 µg du support polyA(+)-ARN et 1 µg/ml de l'amorce spécifique dans la réaction du premier brin de cADN.

L'oligo(dG) "tailing" du cADN, la réaction avec l'oligomère phosphorylé 5'-dAATTCCCCCCCCCCC-3' et la ligation avec le vecteur lambdaNM607 digéré par EcoRI ont été effectués comme cela est décrit dans la référence 9.

L'ADN recombinant ligué a été empaqueté in vitro dans des particules de phage pour infecter un tapis de E. coli POP 101 (réf. 13).

Normalement, 1 ng du cADN oligo(dG)-"tailed" génère $2 \times 10^4$ à $5 \times 10^4$ ufp recombinants.

Des répliques de filtres effectuées à partir de plaques (diamètre : 130 mm) contenant $2 \times 10^4$ ufp sont hybridées une nuit complète dans une solution contenant 6xSSC, 5 % de sulfate de dextrane, 4xsolution de Denhardt, 10 mM de pyrophosphate de sodium, 0,1 % de SDS et l'oligonucléotide synthétique $^{32}$P phosphorylé 18 ou 21 mère. La température d'hybridation est de 42°C pour le 18 mère et 50°C pour le 21 mère.

Les filtres sont lavés dans une solution 6xSSC et 0,1 % de SDS à température d'hybridation appropriée. Les candidats sont répliqués et une seconde sélection est effectuée en utilisant le 18 mère et le 21 mère placés adjacents à ceux utilisés pour le criblage primaire. L'exon 14 (portion 4, ligne pointillée) a été isolé sur un clone lambda unique à partir d'une banque génomique lambdaEMBL3 (réf. 14) ($2 \times 10^6$ phages recombinants)obtenue à partir d'ADN de lymphocytes humains partiellement digérés avec MboI, les tailles étant fractionnées sur gel d'agarose.

Les exons 1 et 2 (portion 5, ligne pointillée) ont été isolés à partir de deux clones séparés d'une banque génomique 4XY dans lambdaEMBL3, obtenue à partir d'une lignée cellulaire lymphoblastoïde humaine GM1202A. Dans les deux cas, deux sondes oligonucléotides 21 mères placés en tandem dans la séquence de FVIII ont été utilisés pour le criblage dans des conditions similaires à celles utilisées pour le criblage des banques de cADN.

Les deux exons ont été sous-clonés dans M13. Ces deux clones ont été linéarisés avec une enzyme de restriction qui coupe dans la séquence intervenante 3' de l'exon 1 et 5' de l'exon 2, traités avec la nucléase Bal31, réparés avec la polymérase de Klenow en présence de 4 dNTPs, digérés avec SstI (clone exon 1) ou EcoRI (clone exon 2) et insérés dans le phage M13TG131 (réf. 15) de façon à ce que les deux exons soient positionnés dans le même vecteur, dans la même orientation correcte, séparés par 1,2 kb de séquence intermédiaire.

Un ADN support mono brin a été préparé à partir de ce clone et la séquence intermédiaire précisément excisée, par élimination de la séquence monobrin sans séquence complémentaire appariée, en utilisant un oligonucléotide 36 mère spécifique pour 18 bases à l'extrémité 3' de l'exon 1 et 18 bases à l'extrémité 5' de l'exon 2.

Des clones correctement mutés ont été identifiés en utilisant l'hybridation du 36 mère à 60°C dans 5xSSC et lavage à 60°C dans 0,2xSSC. Les cADN et les séquences génomiques ont été liés un par un en utilisant les enzymes de restriction indiquées en haut de la figure. Le séquençage de l'ADN de la molécule complètement assemblée a été effectué en utilisant la méthode au didéoxynucléotide (réf. 16) ou la technique de dégradation chimique (réf. 17).

La figure 1 représente schématiquement l'assemblage de la totalité de la région codante de FVIII. Le cADN complet est en haut. Le premier ATG, le codon stop TGA et le site de polyadénylation AATAAA sont indiqués ainsi que les sites de restriction utilisés dans l'assemblage.

Les régions UT 5' et 3' sont hachurées. Les flèches 1, 2 et 3 représentent les clones de cADN utilisés ainsi que leurs amorces. Les séquences génomiques 4 et 5 sont représentées par des lignes pointillées.

En bas de la figure, l'assemblage final est cloné dans le site SalI du plasmide pUC8.

L'analyse de la séquence d'ADN de la région codante révèle deux différences par rapport au FVIII synthétisé par les cellules AL-7 (réf. 1) et par les reins humains (réf. 3) :

1) la position 1035 : A ⟶ G (silencieuse)

2) la position 3864 : A ⟶ C (silencieuse).

La première substitution qui détruit le site unique AccI provient d'une erreur dans le processus de transcription réverse, compte tenu du fait que 14 clones de cADN isolés indépendamment à partir de la même banque maintiennent ce site de restriction.

La seconde substitution est présente dans la portion centrale, qui a été clonée à partir d'une banque génomique et peut représenter un polymorphisme génétique.

EXEMPLE 2

EXPRESSION DU FVIII DANS LES CELLULES DE MAMMIFERES

Dans pTG1080 le site SmaI de pUC8 se trouve en amont de la région 5' UT de FVIII tandis que le site HpaI est présent à la position 7434 de la région 3' UT. Ces deux enzymes ont été utilisées pour exciser la séquence FVIII pour permettre son insertion dans le vecteur pTG186-poly.

Ce plasmide permet la recombinaison homologue
in vivo avec le génome du virus de la vaccine (VV).

L'ADN de différents recombinants VV a été préparé
et analysé par la méthode de Southern pour confirmer
l'intégration de la séquence FVIII dans l'ADN viral (figure 2).

De façon plus précise, les analyses d'hybridation par
Southern des recombinants VV.FVIII ont été effectuées par
digestion avec HindIII (A) ou BamHI (B). Dans chacun de ces
cas, pTG186-poly est le vecteur de recombinaison VV tandis
que pTG1016 et pTG1015 représentent pTG186-poly avec insertion
de la séquence VIII placée soit dans l'orientation correcte,
soit dans l'orientation incorrecte pour la transcription
respectivement. Les clones 10.1 à 10.10 représentent l'ADN
de 10 VV (TK⁻) dérivés après recombinaison entre le génome
de VV et pTG1016. Les clones 6.1 à 6.5 sont dérivés de
pTG1015. Les produits sont hybridés, soit avec (A) un
mélange d'oligonucléotides $^{32}$P phosphorylés (21 mères ou
18 mères) complémentaires de fragments HindIII de la séquence
FVIII, ou (B) avec le FVIII entièrement assemblé (fragment
SmaI-HpaI) marqué au $^{32}$P par "nick translation". Dans la
partie B, seuls les recombinants VV choisis pour des analyses
complémentaires ont été indiqués.

La séquence de FVIII présente dans pUC8 (pTG1080)
a été digéré avec SmaI + HpaI et insérée dans les deux
orientations dans le site BamHI, rendu franc par traitement
à la nucléase $S_1$, de pTG186-poly, un dérivé de pTG186 contenant le polylinker M13TG131 (BglII à EcoRI) (réf. 15). Le
vecteur pTG186-poly peut être obtenu par exemple à partir de
vecteurs décrits dans la demande de brevet WO 85 05376. Le
plasmide pTG186-poly peut être généré à partir du plasmide
pTG188 digéré par PstI et religué par la ligase T4. Le
plasmide pTG393 est mis en digestion avec BamHI pour générer après ligation le plasmide pTG1H-TK-P7,5K ; puis le segment polylinker du bactériophage M13TG131 (réf. 15) est excisé par EcoRI et BglII et inséré entre les sites EcoRI et BamHI du plasmide pTG1H-TK-P7,5K générant pTG186-poly.

Dans cette construction, 10 sites de restriction sont disponibles pour le clonage d'un gène étranger sous le contrôle de P7,5K.

L'insertion de la séquence FVIII dans VV (Copenhagen ts 26) est effectuée comme cela a été décrit (réf. 7) et produit un virus TK⁻.

Après sélection avec la 5-bromodéoxyuridine, l'ADN des recombinants est extrait, découpé avec HindIII ou BamHI, soumis à l'électrophorèse sur gel d'agarose à 1 % et placé sur filtre de nitrocellulose et hybridé avec des sondes tel que décrit précédemment.

Après une nouvelle sélection des clones TK⁻, l'un des recombinants VV contenant l'ADN du FVIII dans une orientation transcriptionnelle correcte (VV.TG.FVIII 10.2-1, dérivé du clone 10.2) a été choisi et utilisé pour infecter différents types de cellule.

Après 1 heure d'absorption, le milieu supplémenté par du sérum de veau foetal (SFC) a été éliminé, les cellules ont été rincées très complètement et un nouveau milieu sans SFC a été utilisé. Après 24 heures, le taux d'antigène FVIII (FVIII.Ag) a été mesuré dans chaque surnageant par dosage immunoradiométrique (IRMA) (réf. 18) et les résultats sont indiqués dans le tableau 1.

# TABLEAU 1

## TAUX D'Ag FVIII (U/ml) DANS LE SURNAGEANT DES CULTURES DE DIFFERENTES LIGNEES CELLULAIRES

| Lignées cellulaires | BHK21 | Vero | HeLa | HepG2 |
|---|---|---|---|---|
| VV.TG.FVIII 10.2-1 | 0,120 | 0,049 | 0,035 | 0,028 |
| WT VV | – | – | – | – |

$2 \times 10^6$ cellules sont infectées à 1 upf/cellule pendant 1 heure. Ensuite le milieu avec SFC est éliminé, les cellules sont lavées puis on les place dans un milieu sans SFC mais contenant 4 % d'albumine de sérum bovin (BSA) et 1 mM $CaCl_2$. Après 24 heures, le titre de FVIII.Ag est mesuré par IRMA, en utilisant une IgG purifiée d'un sérum de patient hémophile inhibiteur comme premier anticorps et un anticorps monoclonal anti-FVIII comme second anticorps.

16

Les surnageants des cellules infectées avec le VV sauvage donnent des résultats insignifiants.

Lorsque les surnageants sont testés pour l'activité pro-coagulante de FVIII (mesure classique du temps partiel de thromboblastine activée - APTT (réf. 19)). des valeurs d'activité très hétérogènes, parfois même sans rapport avec les taux d'antigène correspondants, sont obtenues.

Les cellules BHK21 infectées avec VV.TG.FVIII 10.2-1 sécrètent du FVIII avec un pic d'activité correspondant à environ 0,09 unités (U/ml) à 24 heures, les contrôles étant négatifs. L'activité décroît lentement après 36 à 48 heures, probablement en raison de nombreux facteurs tels que la labilité de la protéine à 37°C (il s'agit là d'un fait qui est déjà connu dans le stockage de concentrés de FVIII plasmatique (réf. 20)) et/ou la lyse des cellules par les VV avec une libération correspondante de protéases intracellulaires et protéolyse progressive de la protéine recombinante.

L'activité pro-coagulante spécifique du FVIII a été inhibée en utilisant un sérum de patients hémophiles inhibiteurs et un anticorps monoclonal anti-FVIII, CAG-1 175A7, (réf. 21) qui neutralise cette activité à différentes dilutions.

Un bruit de fond très important a été trouvé dans les surnageants de cellules Vero de reins de singe et des cellules HeLa infectées avec le virus vaccine sauvage. Ce phénomène peut s'expliquer par une lyse provenant des VV qui conduit à un relarguage de protéases endogènes, qui peuvent interférer avec le test de APTT.

Aucune activité pro-coagulante significative n'a été trouvée dans le surnageant de l'hépatome humain G2 (HepG2) infecté avec VV.TG.FVIII 10.2-1

C'est un résultat qui contraste avec la détection d'un certain taux d'antigène FVIII dans le même échantillon.

17

La question de savoir si le FVIII est sécrété sous forme inactive ou s'il est rapidement inactivé dans ce type de cellule n'est pas encore claire. Ces résultats démontrent qu'un recombinant actif de FVIII est exprimé dans les cellules BHK21.

EXEMPLE 3

STABILISATION DU FVIII

Cette diminution progressive de l'activité pro-coagulante après 24 heures observée dans le cas des cellules BHK21 a conduit la Demanderesse a effectuer des investigations afin de déterminer s'il était possible d'augmenter la stabilité de la molécule recombinante ainsi obtenue.

Il a été ainsi décidé d'utiliser des préparations purifiées de facteur von Willebrand (vWf) (il s'agit d'une glycoprotéine assez grosse qui fonctionne comme une molécule porteuse de FVIII dans le sang et qui joue un rôle actif dans l'adhésion plaquettaire après des dommages vasculaires) (réf. 22).

Après infection des cellules BHK21 avec VV.TG.FVIII 10.2-1 et lavages subséquents, le milieu est supplémenté avec une préparation de vWf (0,5 U/ml).

Les variations de l'activité pro-coagulante sont représentées sur la figure 3 :

● — ● en présence de vWf

O — O en présence d'un autre vWf

■ — ■ sans vWf.

Après une période de 48 heures, l'activité pro-coagulante augmente de façon pratiquement linéaire, le maximum est atteint pour 0,18 U/ml en présence de vWf. En l'absence de vWf, le déclin normal de l'activité peut être noté après 24 heures. Les surnageants de cellules infectées avec le VV sauvage en présence de vWf ne donnent aucune activité pro-coagulante montrant que la contamination par du FVIII dans les préparations de vWf n'est pas significative. Des concentrations de vWf jusqu'à 0,1 U/ml se révèlent efficaces pour stabiliser la protéine recombinante. Des concentrations plus faibles, toutefois, ne protègent plus la molécule de FVIII après 24 heures.

Ces résultats confirment que le recombinant actif FVIII est reconnu et stabilisé par le vWf natif.

Exemple 4    Construction des plasmides portant la séquence codante de la chaîne lourde ou de la chaîne légère du facteur VIII

Le plasmide pTG1016 qui porte la séquence codante complète du facteur VIII a été utilisé pour réaliser les constructions suivantes, qui sont représentées dans la figure 4.

1) pTG1021 : plasmide portant la séquence codante de la chaîne lourde.

Par digestion du plasmide pTG1016 avec l'enzyme SphI, on délète un fragment d'ADN compris entre les nucléotides 3935 et 6585 (numérotation des nucléotides et des acides aminés selon Wood et al. 1). Le plasmide religué porte donc la séquence correspondant aux acides aminés 1 à 1293, c'est-à-dire à la chaîne lourde du facteur VIII, suivie des 144 derniers nucléotides qui restent de la séquence située en aval du deuxième site SphI et d'un codon STOP qui résulte d'un décalage de la lecture des codons ("frameshift") dû à la position du site SphI. (Cette portion d'ADN est quadrillée sur la figure 4).

Ce plasmide est appelé pTG1021.

2) pTG1025 : plasmide portant la séquence codante de la chaîne légère.

Par digestion du plasmide pTG1016 avec l'enzyme BclI, on délète un fragment d'ADN compris entre les nucléotides 402 et 4419. Le plasmide religué porte donc la séquence correspondant au peptide signal, aux acides aminés 1 à 116 de la chaîne lourde puis 1455 à 2232, c'est-à-dire à la chaîne légère, et le codon STOP naturel du gène du facteur VIII.

Ce plasmide est appelé pTG1025.

Exemple 5    Sélection de virus de la vaccine recombinants portant les séquences codantes des chaînes lourde ou légère du facteur VIII des constructions pTG1021 ou pTG1025

Comme dans le plasmide pTG1016 de départ, dans les plasmides pTG1021 et pTG1025, les séquences codantes sont placées sous le contrôle du promoteur de la protéine 7.5 K du virus de la vaccine et l'ensemble de ce bloc fonctionnel d'ADN est inséré dans le gène TK de la vaccine.

Ces plasmides permettent donc l'intégration du bloc fonctionnel d'ADN dans le gène TK d'un virus sauvage de la vaccine, par recombinaison homologue dans les cellules infectées et transfectées par le plasmide, selon une technique devenue classique (décrite notamment dans le brevet français 84.06499).

Le virus sauvage utilisé est le virus de type Copenhagen ts26. La recombinaison est effectuée dans les cellules BHK21. Les virus recombinants sont sélectionnés par leur caractère TK⁻.

Un virus recombinant a été retenu pour chacune des constructions :
- VV.TG.FVIII HC.1 qui dérive du plasmide pTG1021
- VV.TG.FVIII LC.1 qui dérive du plasmide pTG1025
Le virus VV.TG.FVIII 10.2.1 qui porte la séquence complète du facteur VIII et dérive du pTG1016 a été décrit dans l'exemple 2.

20

**Exemple 6**    Synthèse du facteur VIII par les cellules infectées par les virus recombinants et activité
antithrombinique des molécules synthétisées

Dans les deux constructions décrites dans l'exemple
5, les séquences codantes des chaînes HC et LC sont précédées
par la séquence signal naturelle du facteur VIII qui permet
l'excrétion de la molécule dans le milieu de culture. De
plus, les deux séquences comprennent un site de clivage par
la thrombine qui permet l'activation protéolytique de la
molécule.

On peut donc mesurer la présence de la molécule
(c'est-à-dire l'antigène facteur VIII) et son activité dans
le milieu de culture surnageant des cellules.

Des cultures de cellules BHK21 (de $2.10^6$ cellules)
sont infectées avec le virus de la vaccine sauvage, comme
témoin, ou avec les virus recombinants, à une multiplicité
d'infection de 1 ufp/cellule.

Avec les recombinants, 4 inoculations indépendantes
sont effectuées :
- VV.TG.FVIII 10.2.1
- VV.TG.FVIII HC.1
- VV.TG.FVIII LC.1
- VV.TG.FVIII HC.1 + VV.TG.FVIII LC.1, en co-infection.

Après 1 heure d'adsorption, les cultures sont lavées
et le milieu de culture est renouvelé. Ce milieu contient 4 %
d'ASB mais pas de sérum de veau, 1 mM $CaCl_2$ et 0,5 unités/ml
de facteur von Willebrand (vWf). Après 24 ou 48 heures, les
surnageants sont récoltés et immédiatement congelés à l'azote
liquide pour être testés ultérieurement.

1) La présence de molécules de facteur VIII ou de fragments de celui-ci est mesurée par un dosage radioimmunométrique. L'antigène (FVIII:Ag) est dosé "en sandwich" entre de l'immunoglobuline G de sérum de patient hémophile inhibiteur, adsorbée sur la paroi du tube à essai, et un anticorps monoclonal anti-facteur VIII, spécifique d'un épitope de la chaîne L, radioactif (selon une méthode décrite par Lee et al. 19).

Les résultats sont présentés dans le tableau (seule la chaîne L est détectable avec l'anticorps monoclonal utilisé) :

- les cellules infectées avec le recombinant VV.TG.FVIII LC.1 produisent une quantité d'antigène semblable à celle produite par le recombinant VV.TG.FVIII 10.2.1
- les cellules co-infectées par les 2 recombinants LC et HC produisent environ 2 fois moins d'antigène LC, ce qui s'explique par la compétition entre les 2 virus recombinants au niveau de la machinerie cellulaire nécessaire à l'expression des 2 protéines.

2) La mesure de l'activité du facteur VIII (FVIII:C) est réalisée classiquement par le temps partiel de thromboplastine activée (18).

Les résultats sont présentés dans le tableau 2 suivant :

Tableau 2 :   Détermination de la quantité d'antigène FVIII (FVIII:Ag) et de l'activité procoagulante (FVIII:C) dans le surnageant de cultures de cellules BHK21 infectées avec les virus VV recombinants.

| | 24 heures | | 48 heures | |
|---|---|---|---|---|
| | FVIII:C* | FVIII:Ag** | FVIII:C* | FVIII:Ag** |
| VV.TG.FVIII 10.2.1 | 0.140 | 0.142 | 0.235 | 0.470 |
| VV.TG.FVIII HC.1 + VV.TG.FVIII LC.1 | 0.010 | 0.195 | - | 0.095 |
| VV.TG.FVIII HC.1 | - | ND | - | ND |
| VV.TG.FVIII LC.1 | - | 0.407 | - | 0.247 |
| VV sauvage | - | | | |

FVIII:C* (en unités par ml) : déterminé par le temps
        partiel de thromboplastine activée.
FVIII:Ag** (en unités par ml) : déterminé par dosage
        radioimmunométrique.
ND : non déterminé.
-  : sous le seuil de détection.

- dans le surnageant des cellules co-infectées avec
les 2 virus recombinants LC et HC on mesure une
activité procoagulante significative mais plus faible que celle mesurée avec le recombinant FVIII
complet. On note que cette activité décroit après 48
heures, ce qui indique probablement une instabilité
plus grande de la molécule réassociée.
Cette activité est bien spécifique parce qu'elle
peut être neutralisée par l'addition d'anticorps
monoclonal antifacteur VIII ou par l'addition de
sérum de patient hémophile inhibiteur.

- on ne détecte aucune activité FVIII:C dans les surnageants des cellules infectées par le virus sauvage
(contrôle négatif) ni par les recombinants exprimant
seulement la chaîne H ou la chaîne L.

23

Le mélange in vitro de ces surnageants contenant les chaînes H et L ne permet pas de restaurer l'activité procoagulante, soit parce que les quantités produites sont insuffisantes pour permettre la réassociation des 2 chaînes, soit parce que cette réassociation est instable ou ne peut se réaliser que dans des conditions d'environnement particulières.

Le plasmide pTG188 a été déposé à la Collection Nationale de Cultures de Microorganismes de l'Institut Pasteur, 28 rue du Docteur-Roux, 75724 PARIS Cédex 15, le 20 juin 1985 sous le n° E. Coli 5KpTG188 - n° I.458.

Les souches suivantes ont été déposées à la Collection Nationale de Cultures de Microorganismes de l'Institut Pasteur, 28 rue du Docteur-Roux, 75724 PARIS Cédex 15, le 6 juin 1986 :

. Souche E. coli 5K pTG1016 - n° I.558

. Souche E. coli 5K pTG1080 - n° I 559.

## REFERENCES

1. Wood W.I. et al. Nature 312, 330-337 (1984)

2. Toole J.J. et al. Nature 312, 342-347 (1984)

3. Truett M.A. et al. DNA 4, 333-349 (1985)

4. Thorell L. et Blombäck B. Thromb. Res. 35, 431-450 (1984)

5. Smith G.L., Mackett M., Moss B. Nature 302, 490-495 (1983)

6. Panicali D. et al. Proc. Natl. Acad. Sci. USA 80, 5364-5368 (1983)

7. Kieny M-P. et al. Nature 312, 163-166 (1984)

8. Murray N.E., Brammar W.J. et Murray K. Mol. Gen. Genet. 150, 53-61 (1977)

9. Le Bouc Y. et al. FEBS Lett 196, 108-112 (1985)

10. Gitschier J. et al. Nature 312, 326-330 (1984)

11. Chirgwin J.M. et al. Biochemistry 18, 5294-5299 (1979)

12. Pavirani A., Mage R. et Fitzmaurice L. Eur. J. Immunol. 12, 485-490 (1982)

13. Lathe R. et Lecocq J-P. Virology 83, 201-206 (1977)

14. Frischauf A.M. et al. J. Mol. Biol. 170, 827-842 (1983)

15. Kieny M-P., Lathe R. et Lecocq J-P. Gene 26, 91-99 (1983)

16. Sanger F., Nicklen S. et Coulson A.R. Proc. Natl. Sci. USA 74, 5463-5467 (1977)

17. Maxam A.M. et Gilbert W. Methods Enzymol. 65, 499-560 (1980)

18. Girma J-P. et al. Br. J. Haematol. 47, 269-282 (1981)

19. Lee M.L., Maglalang E.A., Kingdon M.S. Thromb. Res. 30, 511-519 (1983)

20. Rock G.A. et al. Thromb. Res. 29, 521-535 (1983)

21. Croissant M-P., Van de Pol H., Lee H.H. et Allain J-P. (en presse)

22. Zimmermann T.S., Meyer D. Hemostasis and Thrombosis, Eds Colman R.W., Hirsch J., Marder V.J. et Salzman E.W. 54-74 (J.B. Lippincot Co. Philadelphia 1982)

23. Vehar, G.A. et al., Nature 312, 337-342 (1984)

24. Fulcher, C.A. et al., J. Clin. Invest. 76, 117-124 (1985)

25. Rotblat, F. et al., Biochemistry 24, 4294-4300 (1985)

26. Eaton, D. et al., Biochemistry 25, 505-512

27. Anderson, L.O. et al. J. Proc. Natl. Acad. Sci. USA 83, 2979-2983 (1986)

28. Fay, P.J. et al., Biochim. Biophys. Acta 871, 268-278 (1986)

29. Pavirani, A. et al. (en presse)

## REVENDICATIONS

1) Procédé de préparation de facteur VIII, caractérisé en ce qu'on met en culture, dans un milieu de culture approprié, des cellules de mammifère infectées par un vecteur assurant l'expression du facteur VIII, ledit milieu contenant le facteur von Willebrand.

2) Procédé selon la revendication 1, caractérisé en ce que le facteur von Willebrand est ajouté à une concentration d'au moins 0,1 U/ml.

3) Procédé selon l'une des revendications 1 et 2, caractérisé en ce que les cellules en culture sont des cellules BHK.

4) Vecteur assurant l'expression du FVIII dans les cellules de mammifère, caractérisé en ce qu'il s'agit d'un poxvirus qui comporte tout ou partie d'une séquence d'ADN codant pour le FVIII.

5) Vecteur selon la revendication 4, caractérisé en ce qu'il s'agit du virus de la vaccine.

6) Vecteur selon l'une des revendications 4 et 5, caractérisé en ce que la séquence d'ADN codant pour le FVIII est sous la dépendance d'un promoteur d'un gène de poxvirus.

7) Vecteur selon la revendication 6, caractérisé en ce que le promoteur est un promoteur d'un gène de la vaccine.

8) Vecteur selon la revendication 6, caractérisé en ce que la séquence d'ADN codant pour le FVIII est sous le contrôle du promoteur du gène de la protéine 7,5K de la vaccine.

9) Vecteur selon l'une des revendications 4 à 8, caractérisé en ce que la séquence codant pour le FVIII est clonée dans le gène TK de la vaccine.

10) Vecteur selon l'une des revendications 4 à 9, caractérisé en ce que la séquence codant pour une partie du facteur VIII code pour au moins l'un des polypeptides, la chaîne lourde du facteur VIII, HC ou la chaîne légère du facteur VIII, LC.

11) Vecteur selon la revendication 10, caractérisé en ce que la séquence en cause code pour la chaîne lourde du facteur VIII.

12) Vecteur selon la revendication 10, caractérisé en ce que la séquence en cause code pour la chaîne légère du facteur VIII.

13) Vecteur selon l'une des revendication 10 à 12, caractérisé en ce que les séquences codant pour HC ou LC sont précédées de la séquence signal naturelle du facteur VIII.

14) Cellule de mammifère infectée par un vecteur selon l'une des revendications 4 à 13.

15) Cellule de mammifère co-infectée par un vecteur selon l'une des revendications 11 ou 13, et par un vecteur selon l'une des revendications 12 ou 13.

16) Cellule de mammifère selon les revendications 14 ou 15, caractérisé en ce qu'il s'agit de cellule BHK21.

17) Procédé de préparation de FVIII caractérisé en ce qu'on cultive des cellules selon l'une des revendications 14 à 16 et que l'on récupère la protéine formée.

18) Procédé de préparation de tout ou partie du facteur VIII, caractérisé en ce qu'on met en culture, dans un milieu approprié, des cellules de mammifère infectées par un vecteur selon l'une des revendications 4 à 13, assurant l'expression du facteur VIII, ledit milieu contenant le facteur von Willebrand.

19) Vecteur selon l'une des revendications 10 à 13, caractérisé en ce qu'il s'agit d'un plasmide comportant une séquence codant pour HC ou LC.

20) Polypeptide ayant la structure de HC ou de LC ou correspondant à la réassociation de HC + LC.

21) Composition thérapeutique comportant à titre de principe actif au moins un polypeptide ou un composé de réassociation selon la revendication 20.

22) FVIII obtenu par la mise en oeuvre du procédé selon la revendication 17.

23) FVIII obtenu à partir de culture cellulaire, caractérisé en ce qu'il est sous forme mature et glycosylée.

FIG.1

E = Eco RI
B = Bam HI
H = Hpa I
P = Pst I
S = Sph I

1Kbase

FIG_2

FIG. 3

0251843

4/4

A)

pTG 1016

pTG 1021

pTG 1025

B)

REGION B

740 — 1649

NH₂ — CHAINE LOURDE ~90 Kd

CHAINE LEGERE ~80 Kd — COOH

B = Bcl I
S = Sph I
H = Hpa I

1 Kbase

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| Y | EP-A-0 162 782 (TRANSGENE S.A.) <br><br> * En entier * | 4-12, 14,15, 17,19 | C 12 N 15/00 <br> C 12 P 21/02 <br> C 12 N 5/00 <br> C 07 K 13/00 <br> A 61 K 37/02 |
| | --- | | |
| X | EP-A-0 150 735 (CHIRON CORP.) <br> * En entier * | 19-23 | |
| Y | | 4-12, 14,17, 19 | |
| | --- | | |
| X | EP-A-0 160 457 (GENENTECH INC.) <br><br> * En entier * | 4-10, 14,17 | |
| Y | | 22,23 | **DOMAINES TECHNIQUES RECHERCHES (Int. Cl.4)** <br><br> C 12 N <br> C 12 P <br> C 07 K <br> A 61 K |
| | --- | | |
| X | WO-A-8 602 838 (NORDISK GENTOFTE) <br> * En entier * | 20,21 | |
| | --- | | |
| X | EP-A-0 182 372 (SCRIPPS CLINIC AND RESEARCH FOUND.) <br> * En entier * | 20-23 | |
| | --- | | |
| P,X | EP-A-0 197 901 (KABIVITRUM) <br> * En entier * | 20-23 | |
| | ---     -/- | | |

Le présent rapport de recherche a été établi pour toutes les revendications

| Lieu de la recherche | Date d'achèvement de la recherche | Examinateur |
|---|---|---|
| LA HAYE | 23-09-1987 | DESCAMPS J.A. |

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| P,X | THE JOURNAL OF BIOLOGICAL CHEMISTRY, vol. 261, no. 27, 25 septembre 1986, pages 12574-12578, The American Society of Biological Chemists, Inc., US; R.L. BURKE et al.: "The functional domains of coagulation factor VIII:C" | 20-23 | |
| P,Y | IDEM | 10-12, 14,15, 17,19 | |
| P,X | BIO/TECHNOLOGY, vol. 5, avril 1987, pages 389-392, New York, US; A. PAVIRANI et al.: "Choosing a host cell for active recombinant factor VIII production using vaccinia virus" * En entier * | 1-10, 14,16, 22,23 | |
| P,X | BIOCHEMICAL AND BIOPHYSICAL RESEARCH COMMUNICATIONS, vol. 145, no. 1, 29 mai 1987, pages 234-240, Academic Press, Inc.; A. PAVIRANI et al.: "Two independent domains of factor VIII co-expressed using recombinant vaccinia viruses have procoagulant activity" * En entier * | 1-23 | |

--- -/-

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 23-09-1987 | DESCAMPS J.A. |

Office européen
des brevets

**RAPPORT DE RECHERCHE EUROPEENNE**

Numero de la demande

EP 87 40 1255

| | **DOCUMENTS CONSIDERES COMME PERTINENTS** | | **Page 3** |
|---|---|---|---|
| Catégorie | Citation du document avec indication, en cas de besoin, des parties pertinentes | Revendication concernée | CLASSEMENT DE LA DEMANDE (Int Cl 4) |
| E | EP-A-0 232 112 (CHIRON CORP.)<br>* En entier * | 20-23 | |
| A | BRITISH JOURNAL OF HAEMATOLOGY,<br>vol. 52, 1982, pages 259-267,<br>Blackwell Scientific<br>Publications; E.G.D. TUDDENHAM<br>et al.: "Response to infusions<br>of polyelectrolyte fractionated<br>human factor VIII concentrate in<br>human haemophilia A and von<br>Willebrand's disease"<br>* Page 265, discussion,<br>paragraphe 1 *<br><br>----- | | |
| | | | **DOMAINES TECHNIQUES RECHERCHES (Int. Cl.4)** |

Le present rapport de recherche a été établi pour toutes les revendications

| Lieu de la recherche | Date d'achèvement de la recherche | Examinateur |
|---|---|---|
| LA HAYE | 23-09-1987 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur. mais publié à la date de dépôt ou après cette date
D : cité dans la demande
L : cité pour d'autres raisons

& : membre de la même famille, document correspondant

OEB Form 1503 03 82